# EUROPEAN PATENT APPLICATION

(11) **EP 1 344 770 A1**
(43) Date of publication of application: **17.09.2003**
(21) Application number: 03251426.7
(22) Date of filing: 10.03.2003
(51) Int. Cl.: C07C 253/10, C07C 255/07, B01J 31/18, C07F 15/04

(54) **Process for the hydrocyanation of butadiene**

(30) Priority: 12.03.2002 US 96799
(71) Applicant: E.I. du Pont de Nemours and Company, Wilmington, DE 19898 (US)
(72) Inventor: McKinney, Ronald James, Wilmington, Delaware 19803 (US)
(74) Representative: Cockerton, Bruce Roger

(57) **Abstract**

This invention is an improved process for the hydrocyanation of butadiene in the presence of a nickel catalyst containing a bidentate-phosphite ligand, wherein the butadiene contains less than 5 ppm (w/w) 4-tert-butylcatechol.

## Description

### BACKGROUND

It is well known in the art that complexes of nickel with phosphorous-containing ligands are useful as catalysts in hydrocyanation reactions. Such nickel complexes using monodentate phosphites are known to catalyze hydrocyanation of butadiene to produce a mixture of C5 nitriles. The desirable products obtained from this reaction are shown by formulas A to C, 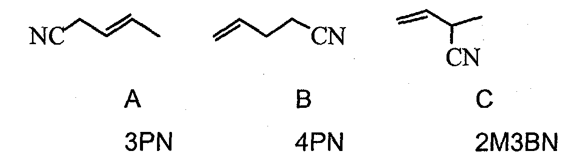 in which 3PN denotes 3-pentenenitrile, 4PN is 4-pentenenitrile, and 2M3BN is 2-methyl-3-butenenitrile.

These catalysts are also useful in the isomerization of 2-methyl-3-butenenitrile to 3-pentenenitrile, and subsequent hydrocyanation of pentenenitriles to produce adiponitrile, an important intermediate in the production of nylon.

It is also well known that most commercially available butadiene contains 4-tert-butylcatechol (TBC) in amounts ranging from 10-300 ppm, which is introduced by the butadiene supplier as an inhibitor against peroxidation and polymer formation, which are undesirable reactions that can lead to vessel rupture and explosion.

TBC is considered undesirable in hydrocyanation reactions because of the tendency of the TBC to degrade catalyst performance. However, small amounts of TBC can be tolerated in the hydrocyanation of butadiene with catalysts comprising nickel and the so-called monodentate phosphite ligands, because such hydrocyanation reactions typically involve high concentrations of ligand.

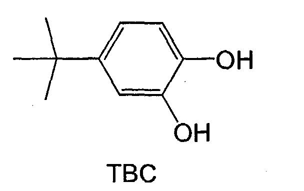

In recent years, a new class of hydrocyanation ligands has been described. This class of ligands is generally denoted as "bidentate ligands" and includes diphosphites, diphosphonites, and diphosphinites. It is further known that these bidentate ligands can also be used to form nickel-based catalysts to perform such hydrocyanation reactions. Generally, theses ligands and catalysts are used in much smaller concentrations than the monodentate phosphite ligands based nickel catalyst, making ligand degradation by TBC more detrimental to catalyst performance in the hydrocyanation or isomerization reactions described above. Accordingly, there is a need for a butadiene hydrocyanation process in which bidentate ligands are protected from TBC-induced degradation.

### SUMMARY OF THE INVENTION

A process for hydrocyanating butadiene, comprising contacting butadiene containing less than 5 ppm (weight to weight) 4-tert-butylcatechol with HCN in the presence of a catalyst comprising nickel and a bidentate phosphorus-containing ligand at a temperature in the range of 25 degrees C to 200 degrees C and a pressure in the range of 5 kPa to 10,000 kPa, and wherein the molar ratio of butadiene to catalyst is from 10:1 to 100,000:1 and the molar ratio of HCN to catalyst is from 10:1 to 100,000:1.

### DETAILED DESCRIPTION OF THE INVENTION

The present invention involves the discovery that the process for hydrocyanating butadiene with nickel catalysts containing bidentate-phosphite ligands is substantially improved when TBC is removed from the butadiene prior to the hydrocyanation reaction. The presence of TBC in the butadiene is believed to be detrimental to the process for the following reasons:
1. TBC reacts with bidentate phosphite ligands producing new monophosphite species. Bidentate-phosphite ligands are significantly more expensive to produce than monodentate-phosphite ligands, and therefore loss of ligand by this mechanism may create an unacceptable economic burden which can make the process unattractive;
2. The monophosphite species so produced react with the nickel catalyst to produce a new nickel complex (A) which contains both the bidentate phosphite and the TBC-derived monodentate phosphite and is inactive for hydrocyanation; in this manner, a second bidentate-phosphite is deactivated and lost for catalytic activity; and
3. The new nickel complex (A) is recovered with the active catalyst and slowly builds up as an inert mass in the catalytic system and decreases reaction volume.

Though similar reactions occur between TBC and monodentate-phosphite ligands, typical process concentrations of monodentate-phosphite ligands are much higher than for bidentate-phosphite ligands, and such losses previously have been accepted because these ligands are relatively inexpensive and the proportion of ligand degraded in this manner is small. However, because bidentate-phosphite ligands are utilized in such low concentrations, the proportion of ligand degraded in this manner can be an order of magnitude higher.

TBC may be removed from butadiene by a variety of techniques, including vaporization (flashing or distillation; the TBC is non-volatile) or passing the liquid butadiene over an absorbant bed such as alumina. The former is practiced commercially on a large scale.

It is also advantageous to minimize other impurities in the butadiene.
1. The amount of vinylcyclohexene in the butadiene should be minimized because it can form azeotropes with one of the desired products during separation of the products from the catalyst leading to a yield loss. Vinylcyclohexene is an impurity in butadiene arising from thermally-induced dimerization of the butadiene. It may be removed from the butadiene by distillation.
2. The amount of butadiene-peroxide, a polymeric peroxide arising from the reaction of butadiene with oxygen, should be minimized because it can destructively oxidize both nickel catalyst and bidentate-phosphite ligand.
3. The amount of vinylacetylene in butadiene should be minimized because it inhibits catalyst activity.

Nickel is combined with a bidentate-phosphite ligand of the present invention to provide the hydrocyanation catalyst. A zero-valent nickel compound that contains a ligand that can be displaced by the bidentate-phosphite ligand of the present invention is the most preferred source of Group VIII metal or Group VIII metal compound. Zero-valent nickel compounds can be prepared or generated according to methods known in the art, such as those described in U.S. Patents 3,496,217; 3,631,191; 3,846,461; 3,847,959 and 3,903,120, incorporated herein by reference. A preferred zero-valent nickel compounds is Ni(COD)₂ (COD is an abbreviation for 1,5-cyclooctadiene). Alternatively, divalent nickel compounds can be combined with a reducing agent, to serve as a source of zero-valent nickel in the reaction. Suitable divalent nickel compounds include compounds of the formula NiZ²₂ where Z² is halide, carboxylate, or acetylacetonate. Suitable reducing agents include metal borohydrides, metal aluminum hydrides, metal alkyls, Li, Na, K, Zn, Fe, or H₂. Elemental nickel, preferably nickel powder, when combined with a halogenated catalyst, as described in U.S. Patent 3,903,120 (incorporated herein by reference), is also a suitable source of zero-valent nickel.

Suitable ligands for the present invention are bidentate phosphorus (III)-containing ligands selected from the group consisting of bidentate phosphites, bidentate phosphinites, and bidentate phosphines. Most preferred ligands are bidentate phosphite ligands.

The most preferred bidentate phosphite ligands are of the following structural formulae:

(R¹O)₂P(OZO)P(OR¹)₂,

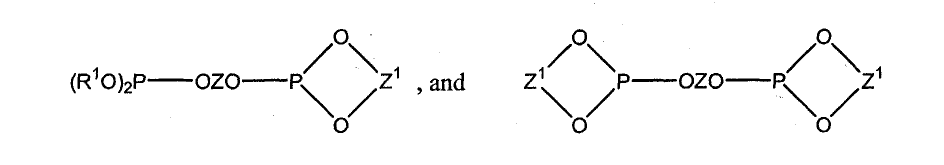

In these formulae, R¹ is phenyl, unsubstituted or substituted with one or more C₁ to C₁₂ alkyl or C₁ to C₁₂ alkoxy groups; or naphthyl, unsubstituted or substituted with one or more C₁ to C₁₂ alkyl or C₁ to C₁₂ alkoxy groups; and Z and Z¹ are independently selected from the groups consisting of structural formulae I, II, III, and IV: 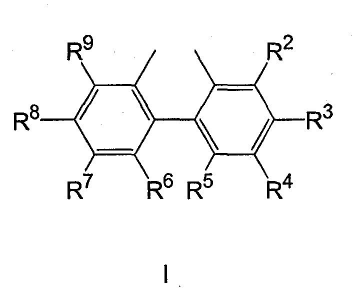 wherein:
R² and R⁹ are the same and are selected from H, C₁ to C₁₂ alkyl, and C₁ to C₁₂ alkoxy;
R³ and R⁸ are the same and are selected from H, C₁ to C₁₂ alkyl, and C₁ to C₁₂ alkoxy;
R⁴ and R⁷ are the same and are selected from H, C₁ to C₁₂ alkyl, and C₁ to C₁₂ alkoxy;
R⁵ and R⁶ are the same and are selected from H, C₁ to C₁₂ alkyl, and C₁ to C₁₂ alkoxy; 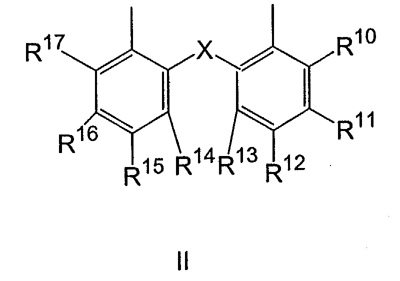
wherein:
X is O, S, or CH(R¹⁸);
R¹⁰ and R¹⁷ are the same and are selected from H, C₁ to C₁₂ alkyl, and C₁ to C₁₂ alkoxy;
R¹¹ and R¹⁶ are the same and are selected from H, C₁ to C₁₂ alkyl, and C₁ to C₁₂ alkoxy;
R¹² and R¹⁵ are the same and are selected from H, C₁ to C₁₂ alkyl, and C₁ to C₁₂ alkoxy;
R¹³ and R¹⁴ are the same and are selected from H, C₁ to C₁₂ alkyl, and C₁ to C₁₂ alkoxy; and
R¹⁸ is H or C₁ to C₁₂ alkyl; 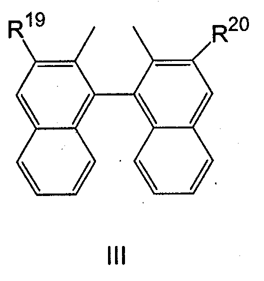
wherein:
R¹⁹ and R²⁰ are the same and are selected from H, C₁ to C₁₂ alkyl, and C₁ to C₁₂ alkoxy; and CO₂R²¹,
R²¹ is C₁ to C₁₂ alkyl or C₆ to C₁₀ aryl, unsubstituted or substituted with C₁ to C₄ alkyl groups. The aryl groups are preferably phenyl or naphthyl. 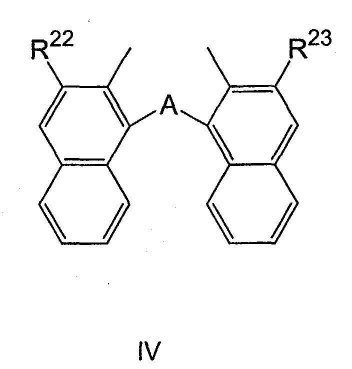
wherein:
A is O, S, CH(R²⁴);
R²² and R²³ are the same and are selected from H and CO₂R²⁵;
R²⁴ is H or C₁ to C₁₂ alkyl;
R²⁵ is C₁ to C₁₂ alkyl; or
In the above structural formulae, the C₁ to C₁₂ alkyl, and C₁ to C₁₂ alkoxy groups may be straight chains or branched.

Examples of ligands suitable for this process are those described in U. S. Pat. Nos. 5,512,695; 5,512,696; 5,663,369; 5,688,986; 5,723,641; 5,847,191; 5,981,772; 6,020,516; 6,127,567; 6,171,997; incorporated herein by reference.

The hydrocyanation process can be carried out, for example, by charging a suitable vessel such as a reactor with butadiene, catalyst composition, and solvent, if any, to form a reaction mixture. Hydrogen cyanide can be initially combined with other components to form the mixture. However, it is preferred that HCN be added slowly to the mixture after other components have been combined. Hydrogen cyanide can be delivered as a liquid or as a vapor to the reaction. As an alternative, a cyanohydrin can be used as the source of HCN. See, for example, U.S. Patent 3,655,723, incorporated herein by reference.

Hydrocyanation processes may also be improved by minimizing the amount of harmful impurities in the HCN. Thus, the hydrocyanation of butadiene with a nickel catalyst containing a bidentate-phosphite ligand is more effective with HCN containing less than 50 ppm of sulfur dioxide or, preferably, less than 10 ppm of sulfur dioxide; less than 50 ppm of sulfuric acid, or, preferably, less than 10 ppm of sulfuric acid; less than 100 ppm cyanogen, or, preferably, less than 20 ppm of cyanogen; less than 50 ppm epoxide, or, preferably, less than 10 ppm of epoxide; less than 100 ppm acrylonitrile, or, preferably, less than 20 ppm of acrylonitrile; less than 5 ppm carbon monoxide, or preferably less than 1 ppm carbon monoxide; less than 100 ppm water, or, preferably less than 20 ppm water; less than 5 ppm oxygen, or, preferably less than 1 ppm oxygen; or mixture thereof.

Another suitable technique is to charge the vessel with the catalyst and the solvent (if any) to be used, and feed both the unsaturated compound and the HCN slowly to the reaction mixture.

The molar ratio of butadiene to catalyst can be varied from about 10:1 to about 100,000:1, preferably 100:1 to 5,000:1. The molar ratio of HCN to catalyst generally is varied from about 10:1 to 100,000:1, preferably 100:1 to 5,000:1.

Preferably the reaction mixture is agitated. For example, by stirring or shaking. A commercial plant might provide mixing through draft tubes, inert gas, etc. The reaction product can be recovered by conventional techniques such as distillation. The reaction can be run either batchwise or continuously.

The hydrocyanation of butadiene can be carried out with or without a solvent. The solvent, if used, can be liquid at the reaction temperature and pressure and inert towards the unsaturated compound and the catalyst. Suitable solvents include hydrocarbons such as benzene, xylene, or combinations thereof; ethers such as tetrahydrofuran (THF); nitriles such as acetonitrile, benzonitrile, adiponitrile, or combinations of two or more thereof. Butadiene can itself serve as the solvent.

The exact temperature is dependent to a certain extent on the particular catalyst being used, and the desired reaction rate. Normally, temperatures of from 25°C to 200°C can be used, the range of 50°C to 150°C being preferred.

The process is generally carried out at a pressure sufficient to keep the butadiene dissolved in the reaction mixture at the reaction temperature. Hence pressures of from about 5 to 20 atmospheres (about 500 to 2000 kPa) are preferred. Higher pressures, up to 10,000 kPa or more, can be used, if desired, but any benefit that may be obtained thereby would probably not justify the increased cost of such operations.

The time required can be in the range of from a few seconds to many hours (such as 2 seconds to 24 hours), depending on the particular conditions and method of operation.

## Claims

1. A process for hydrocyanating butadiene, comprising contacting butadiene containing less than 5 ppm (weight to weight) 4-tert-butylcatechol with HCN in the presence of a catalyst comprising nickel and a bidentate phosphorus-containing ligand at a temperature in the range of 25 degrees C to 200 degrees C and a pressure in the range of 5 kPa to 10,000 kPa, and wherein the molar ratio of butadiene to catalyst is from 10:1 to 100,000:1 and the molar ratio of HCN to catalyst is from 10:1 to 100,000:1.
